# EUROPÄISCHE PATENTSCHRIFT

(11) **EP 2 981 815 B1**
(45) Veröffentlichungstag und Bekanntmachung des Hinweises auf die Patenterteilung: **19.04.2017**
(21) Anmeldenummer: 14724624.3
(22) Anmeldetag: 11.03.2014
(51) Int. Cl.: G01N 27/18, G01K 15/00

(54) **VORRICHTUNG FÜR DIE MESSUNG DER WÄRMELEITFÄHIGKEIT VON GASKOMPONENTEN EINES GASGEMISCHES**
DEVICE FOR MEASURING THE THERMAL CONDUCTIVITY OF GAS COMPONENTS OF A GAS MIXTURE
DISPOSITIF PERMETTANT DE MESURER LA CONDUCTIBILITÉ THERMIQUE DE COMPOSANTS GAZEUX D'UN MÉLANGE GAZEUX

(30) Priorität: 05.04.2013 DE 102013103388
(43) Veröffentlichungstag der Anmeldung: 10.02.2016
(73) Patentinhaber: Chemec GmbH, 33607 Bielefeld (DE)
(72) Erfinder: BRANDAU, Eckard, 33330 Gütersloh (DE)
(74) Vertreter: Brandt, Detlef
(86) Internationale Anmeldenummer: PCT/DE2014/000110
(87) Internationale Veröffentlichungsnummer: WO 2014/161521

(56) Entgegenhaltungen:
- DE-A1- 1 926 250
- DE-A1- 19 639 627
- JP-A- 2001 050 943
- US-A- 4 164 862

## Beschreibung

### Technisches Umfeld

Die Erfindung betrifft eine Vorrichtung für die Messung der Wärmeleitfähigkeit von Gaskomponenten eines Gasgemisches zur Bestimmung der Anteile der Gaskomponenten mit einer Mehrzahl von Wärmeleitfähigkeitssensoren, wobei jeder Wärmeleitfähigkeitssensor Bestandteil einer Widerstandsbrückenschaltung ist und mit einer zur Vorrichtung gehörenden Auswerteeinheit verbunden ist.

### Stand der Technik

In der Gasanalysetechnik gibt es unterschiedliche Methoden, die Gaskonzentrationen der Bestandteile von Gasgemischen zu ermitteln. JP 2001 050943 A, DE 196 39 627 A1 und US 4 164 862 A beschreiben Vorrichtungen zur Messung der Zusammensetzung von Gasgemischen.

Verbreitet ist beispielsweise das Verfahren der Gaschromatographie. Hier wird das zu prüfende Gasgemisch in eine von einem Trägergas durchströmte Trennsäule eingespritzt. Die Substanzen des Gasgemisches werden durch das Trägergas in der Trennsäule transportiert, wobei durch eine konstante Temperatur innerhalb der Trennsäule oder durch eine kontrollierte Temperaturerhöhung eine schnelle und weitgehende Trennung des Gasgemisches herbeigeführt wird. Am Ausgang der Trennsäule wird mittels eines Detektors ermittelt, wenn eine der Substanzen des Gasgemisches die Trennsäule verlässt. Das vom Detektor ermittelte elektronische Signal kann als Pik registriert werden und es kann aus diesem Signal mit entsprechender Auswertungssoftware die Zusammensetzung des Gasgemisches ermittelt werden. Messaufbau und Verfahrensdurchführung sind bei der Gaschromatographie sowohl in zeitlicher als auch konstruktiver Hinsicht aufwändig.

Neben der Gaschromatographie ist es allgemein bekannt, zur Konzentrationsbestimmung einzelner Gase eines Gasgemisches die Unterschiede in der Wärmeleitfähigkeit der beteiligten Gase zu nutzen.

Prinzipiell wird ein als Wärmequelle dienendes Widerstandsheizelement mittels Stromdurchfluss auf eine gegenüber seiner Umgebung erhöhte Temperatur gebracht. Über eine durch geometrische Rahmenbedingungen festgelegte Wärmeleitstrecke wird vom zu prüfenden Gas Wärme von dem Widerstandsheizelement als Wärmequelle zu einer auf konstanter Temperatur gehaltenen Wärmesenke geleitet. Durch den Wärmetransport vom Widerstandsheizelement zur Wärmesenke wird der Wärmequelle Energie entzogen, die ein Maß für die Wärmeleitfähigkeit des Gases darstellt und die sich mit geeigneten Verfahren messen lässt.

Das Verfahren zur Ermittlung der Gaskonzentrationen von Gasgemischen mittels der Wärmeleitfähigkeit dieser Gase erfolgt für die verschiedenen Gaskomponenten mit Hilfe unterschiedlicher Temperaturen des Widerstandsheizelementes.

Zur Messung der Gaskonzentration können zum einen eine Mehrzahl von Wärmeleitfähigkeitssensoren entsprechend der Anzahl der Gaskomponenten eingesetzt werden. Alternativ hierzu ist die Verwendung eines Wärmeleitfähigkeitssensors denkbar, bei dem zeitabhängig in bestimmten Intervallen das Widerstandsheizelement auf unterschiedliche Temperaturen aufgeheizt wird.

Schwierigkeit bei allen mit Wärmeleitfähigkeitssensoren arbeitenden Systemen ist es, dass diese mit Hilfe eines Testgases bekannter Zusammensetzung überprüft und gegebenenfalls kalibriert werden müssen. Diese Kalibrierung muss in regelmäßigen Abständen wiederholt werden, um sicherzustellen, dass die Messvorrichtung insgesamt zuverlässig und richtig arbeitet. Daher ist bei den Nutzern einer derartigen Vorrichtung für die Messung der Wärmeleitfähigkeit von Gaskomponenten eines Gasgemisches immer eine ausreichende Versorgung mit Testgas sicherzustellen. Dies stößt insbesondere außerhalb Europas und Amerikas auf Schwierigkeiten, da nur in den betreffenden geographischen Bereichen für die Kalibrierung notwendige Testgase hergestellt werden. Zoll-, Transport- und Sicherheitsvorschriften erschweren den Transport in Länder des asiatischen und afrikanischen Raumes, wobei auch zu beachten ist, dass die für den Transport der Testgase verwendeten Behältnisse durchaus auch für terroristische Zwecke missbraucht werden können.

### Aufgabe der Erfindung

Ausgehend von den geschilderten Nachteilen der unterschiedlichen Verfahren zur Ermittlung von Gaskonzentrationen der Bestandteile eines Gasgemisches besteht die Aufgabe der Erfindung darin, eine Vorrichtung der eingangs geschilderten gattungsgemäßen Art bereitzustellen, bei der zum Einen auf die Verwendung eines Testgases verzichtet werden kann, die darüber hinaus einfach und somit preisgünstig aufgebaut ist und die durch ihren Aufbau keine Spülphasen bei der Messung mehrerer Gasbestandteile mittels eines einzelnen Wärmeleitfähigkeitssensors mehr erfordert.

### Lösung der Aufgabe

Die gestellte Aufgabe wird in Zusammenschau mit den gattungsbildenden Merkmalen des Anspruches 1 durch die im kennzeichnenden Teil des Hauptanspruches offenbarten technischen Merkmale gelöst.

Erfindungswesentlich dabei ist es, dass jeder Wärmeleitfähigkeitssensor der Vorrichtung ein Heizelement und ein integriertes Temperaturmesselement aufweist, welche bei einer Temperaturänderung des Wärmeleitfähigkeitssensors infolge einer Wärmeabfuhr durch das Gasgemisch zwei Messspannungen generieren, die in der Auswerteeinheit zur Ermittlung von Messfehlern verglichen werden.

Die neuartige konstruktive Gestaltung der Vorrichtung ermöglicht es nunmehr, nach einer Erstkalibrierung im Produktionsbetrieb nach Herstellungsabschluss die ordnungsgemäße Arbeitsweise der erfindungsgemäßen Vorrichtung kontinuierlich zu überwachen. Sämtliche Messfehler, die sich aufgrund des Aufbaus der Vorrichtung ergeben könnten, wie beispielsweise eine Drift des Heizelementes oder des Temperaturmesselementes sowie der Ausfall eines der zur Brückenschaltung gehörenden Widerstände lassen sich durch die Auswertung der generierten Messspannungen zuverlässig ermitteln.

Es ist somit nicht länger notwendig, die korrekte Arbeitsweise der einzelnen zur erfindungsgemäßen Vorrichtung gehörenden Wärmeleitfähigkeitssensoren durch regelmäßige Überprüfung mit einem in seiner Zusammensetzung bekannten Testgas sicherzustellen. Somit entfallen alle in Verbindung mit dem Testgas notwendigen Herstell-, Transport- und Prüfkosten.

Durch den integrierten Aufbau jedes Wärmeleitfähigkeitssensors ergibt sich ein robuster und platzsparender Aufbau der erfindungsgemäßen Vorrichtung, so dass auf alle bislang notwendigen zusätzlichen Komponenten für die Testgaskalibrierung wie beispielsweise Magnetventile, Pumpenfilter, Schläuche und Verschraubungen verzichtet werden kann. Die neuartige Vorrichtung lässt sich somit direkt in eine entsprechende Gasleitung implizieren, wobei durch die Wartungsfreiheit der Vorrichtung sich die Zuverlässigkeit der Gasanalyse signifikant erhöht.

Besondere Ausgestaltungen des Gegenstandes der Erfindung ergeben sich zusammen mit der technischen Lehre des Anspruches 1 zusätzlich aus den Merkmalen der auf den Hauptanspruch rückbezogenen Unteransprüche.

Im Hinblick auf einen robusten und wartungsfreien Aufbau der erfindungsgemäßen Vorrichtung hat es sich insbesondere als vorteilhaft erwiesen, das Heizelement als PT20-Sensor auszubilden und für das Temperaturmesselement einen PT100-Sensor zu verwenden. Die in Rede stehenden Elemente lassen sich problemlos zu einem Wärmeleitfähigkeitssensor zusammenfassen. Der Aufbau des Wärmeleitfähigkeitssensors ist dabei vorteilhafterweise auf einem zentralen Keramikkörper vornehmbar, der beispielsweise aus Siliziumoxyd besteht. Auf diesem Keramikkörper werden mittels eines Dünnschichtverfahrens im Siebdruck Leiterbahnen aus einer platinhaltigen Paste aufgebracht, die den PT20- und den PT100-Sensor bilden. Zum Schutz der Platinleiterbahnen auf dem Keramikkörper wird dieser mit einem Glasüberzug versehen, so dass chemische Reaktionen zwischen der Platinpaste und eventuell zu detektierenden aggressiven Komponenten eines Gasgemisches zuverlässig ausgeschlossen werden.

Hinsichtlich des elektrischen Aufbaues der erfindungsgemäßen Vorrichtung kann es darüber hinaus vorteilhaft sein, jeden Leitfähigkeitssensor mit einem Temperaturspannungswandler zu versehen. Ergänzt wird der elektrische Aufbau zusätzlich durch einen Differenzverstärker, der die innerhalb der Brückenschaltung ermittelte Brückenspannung in ein massebezogenes Signal umwandelt.

### Figurenbeschreibung

Nachfolgend wird der erfindungsgemäße Aufbau der Vorrichtung anhand der beigefügten Zeichnungen näher erläutert. Es zeigt:
- Figur 1: eine schematische Darstellung des Aufbaus eines zur erfindungsgemäßen Vorrichtung gehörenden Wärmeleitfähigkeitssensors mit nachgeschalteten elektronischen Bauteilen und
- Figur 2: die Ansicht eines Wärmeleitfähigkeitssensors, mit dem eine erfindungsgemäße Vorrichtung aufgebaut werden kann.

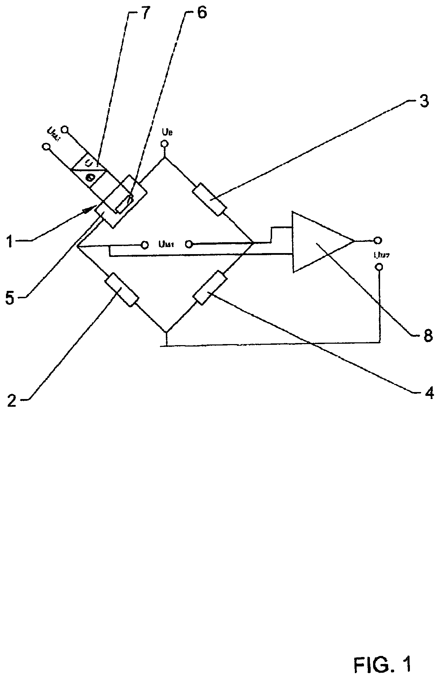

Das in der Figur 1 abgebildete Schemaschaltbild zeigt den Aufbau eines Wärmeleitfähigkeitssensors, mit dem eine Komponente eines Gasgemisches in ihrer Konzentration messbar ist.

Die Gesamtvorrichtung für die Messung der Leitfähigkeit von Gaskomponenten eines Gasgemisches zur Bestimmung der Anteile der Gaskomponenten besteht für N-Anteile aus N-Wärmeleitfähigkeitssensoren. Den Wärmeleitfähigkeitssensoren zugeordnet ist eine zentrale Auswerteeinheit, in der die von den einzelnen Wärmeleitfähigkeitssensoren ermittelten Messspannungen zugeordnet, verglichen und ausgewertet werden.

Auf die Darstellung der Auswerteeinheit wurde in der Zeichnung verzichtet, da sie nicht vorrangig zum erfindungswesentlichen Teil der Vorrichtung gehört.

Der prinzipielle Aufbau eines jeden Wärmeleitfähigkeitssensors besteht aus einer Widerstandsbrückenschaltung, bei der anstelle eines Widerstandes R1 ein Wärmeleitfähigkeitssensor angeordnet ist. Dieser Wärmeleitfähigkeitssensor besteht aus einem Heizelement, beispielsweise in Form eines PT20-Sensors und einem Temperaturmesselement, beispielsweise in Form eines PT100-Sensors als integralem Bauteil des Wärmeleitfähigkeitssensors, der in seiner Gesamtheit mit 1 bezeichnet ist. Die übrigen Widerstände sind mit 2, 3 und 4 gekennzeichnet.

Dem Wärmeleitfähigkeitssensor 1 zugeordnet ist ein Temperatur/Spannungswandler 7, welcher eine Messspannung U_{M3} generiert. Die Brückenschaltung wird während des Betriebes der erfindungsgemäßen Vorrichtung mit einer Spannung U_{B} beaufschlagt. Die Spannung U_{B} bewirkt ein Aufheizen des Heizelementes 5 auf eine im Rahmen der Herstellung durch Kalibrierung festgelegte Temperatur T_{Heizer}. Die Temperatur T_{Heizer} ist für jeden Wärmeleitfähigkeitssensor der erfindungsgemäßen Vorrichtung dem zu messenden Gasbestandteil des Gasgemisches angepasst und somit unterschiedlich.

Die Temperatur des Heizelementes 5 wird mittels des Temperaturmesselementes 6 gemessen und durch die Messspannung U_{M3} ausgegeben. Während des Betriebes der erfindungsgemäßen Vorrichtung wird ein Teil der vom Heizelement 5 erzeugten Wärme über das Messgas, welches den Wärmeleitfähigkeitssensor überstreicht, abgeführt. Je höher die Wärmeleitfähigkeit des überstreichenden Gases ist, d.h. umso mehr Wärme abgeführt wird, umso kühler ist die Oberfläche des Heizelementes 5. Proportional zur Wärmeleitfähigkeit des Gases ändert sich die Brückenspannung U_{M1}, die mit dem nachfolgenden Differenzverstärker 8 in eine Masse bezogene Messspannung U_{M2} umgewandelt wird.

Die gesamte Vorrichtung arbeitet korrekt, solange sich die Spannungen U_{M2} und U_{M3} im gleichen Verhältnis zueinander ändern. Dies wird mittels der Auswerteeinheit kontinuierlich überwacht. Somit überprüft sich die erfindungsgemäße Vorrichtung permanent selbst.

Tritt zwischen den beiden Messspannungen eine Differenz auf, liegt eine Fehlfunktion der Vorrichtung vor. Somit kann auf die regelmäßige Kalibrierung und Prüfung mittels eines Testgases verzichtet werden.

Eine Fehlfunktion kann unterschiedliche Ursachen haben:
1. Drift der Brückenspannung U_{B}
   Mittels eines Präzisions-AD-Wandlers werden die jeweiligen Brückenspannungen über einen Multiplexer permanent überprüft und entsprechend nachgeregelt.
2. Drift des Heizelementes 7
   Sollte dieser Fehler auftreten, wird das Heizelement mit Umgebungsluft beaufschlagt und die Brückenspannung U_{B} soweit nachgeregelt, dass die Grundtemperatur T_{Heizer} bei Luft der abgespeicherten Grundtemperatur in der Software der Auswerteeinheit entspricht.
3. Drift des Temperaturmesselementes
   Für den Fall, dass das Temperaturmesselement 6 driftet, verändert sich der entsprechende Temperaturkoeffizient des Temperaturmesselementes 6. Zur Überprüfung wird der Wärmeleitfähigkeitssensor mit Umgebungsluft beaufschlagt und die Grundtemperatur T_{Heizer} gemessen. Anschließend werden alle Betriebsspannungen ausgeschaltet, so dass jeder der zur Vorrichtung gehörenden Heizelemente der Wärmeleitfähigkeitssensoren auf Raumtemperatur abkühlen. Alle Temperaturmesselemente müssen dann die gleiche Temperatur anzeigen. Sofern sich die Differenzen bei der Grundtemperatur unter der Umgebungstemperatur durch iterative Änderung des Nullpunktes und der Verstärkung des Temperaturspannungswandlers 7 ausgleichen lassen, kann die erfindungsgemäße Vorrichtung weiterverwendet werden. Sollte dies nicht der Fall sein, ist die gesamte Vorrichtung auszutauschen.
4. Drift des Temperaturspannungswandlers 7
   Eine derartige Fehlfunktion kann mit Hilfe einer Präzisionsdiode überprüft und gegebenenfalls nachjustiert werden.
5. Drift des Differenzverstärkers 8
   Eine derartige Fehlfunktion ist eindeutig erkennbar und kann durch Nachjustierung des Differenzverstärkers 8 bei Umgebungsluft ausgeglichen werden.Sollte in seltenen Fällen der Ausfall eines Widerstandes 2, 3 oder 4 zu verzeichnen sein, so ist der entsprechende Wärmeleitfähigkeitssensor ebenfalls auszutauschen.

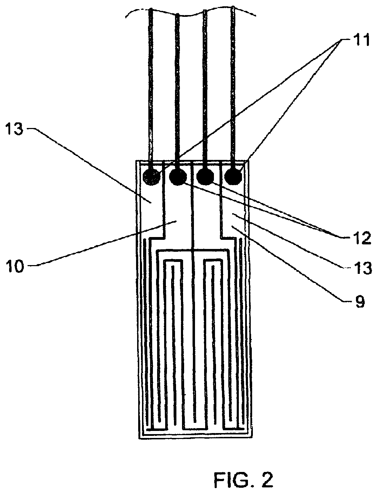

Der praktische Aufbau eines zur erfindungsgemäßen Vorrichtung gehörenden Wärmeleitfähigkeitssensors 1 ist in der Abbildung 2 verdeutlicht. Die Abbildung zeigt einen keramischen Grundkörper 9, der wesentliches Element des Wärmeleitfähigkeitssensors ist. Der Grundkörper kann vorteilhafterweise aus Siliziumoxyd bestehen und ist an seiner Oberseite mit Leiterbahnen 10 aus einer platinhaltigen Paste beschichtet. Die Trennung der Leiterbahnen 10 ist in der Figur 2 durch schwarze Trennstriche verdeutlicht.

Wie aus der Abbildung ersichtlich ist, besitzt der Grundkörper 9 zwei Anschlusspunkte 11 und zwei Anschlusspunkte 12, die jeweils zu einer Leiterbahn 10 bzw. 13 gehören. Der dargestellte Grundkörper besitzt eine Grundfläche von 5 x 2 mm und kann somit problemlos auch bei Verwendung mehrerer Wärmeleitfähigkeitssensoren in eine Gasleitung eingebaut werden. Weitergehende Elemente für die Messung der Wärmeleitfähigkeit einzelner Gaskomponenten eines Gasgemisches zur Bestimmung der Anteile der Gaskomponenten sind im Gegensatz zu den herkömmlichen Messvorrichtungen nicht notwendig. Die Auswerteeinheit kann getrennt aufgebaut werden.

Des weiteren ist festzustellen, dass die gleichzeitige Verwendung von N-Wärmeleitfähigkeitssensoren für N-Anteile eines Gasgemisches Spülphasen zwischen den Messungen einzelner Bestandteile, wie sie bei einigen herkömmlichen Messvorrichtungen notwendig sind, entfallen kann.

### Bezugszeichenliste:

- 1: Wärmeleitfähigkeitssensor
- 2: Widerstand
- 3: Widerstand
- 4: Widerstand
- 5: Heizelement
- 6: Temperaturmesselement
- 7: Temperatur/Spannungswandler
- 8: Differenzverstärker
- 9: Grundkörper
- 10: Leiterbahn
- 11: Anschlusspunkt
- 12: Anschlusspunkt
- 13: Leiterbahn

## Patentansprüche

1. Vorrichtung für die Messung der Wärmeleitfähigkeit von Gaskomponenten eines Gasgemisches zur Bestimmung der Anteile der Gaskomponenten mit einer Mehrzahl von Wärmeleitfähigkeitssensoren (1), wobei jeder Wärmeleitfähigkeitssensor (1) Bestandteil einer Widerstandsbrückenschaltung ist und mit einer zur Vorrichtung gehörenden Auswerteeinheit verbunden ist, wobei jeder Wärmeleitfähigkeitssensor (1) ein Heizelement (5) und ein integriertes Temperaturmesselement (6) aufweist, **dadurch gekennzeichnet, dass** das Heizelement und das integrierte Temperaturmesselement dazu konfiguriert sind, bei einer Temperaturänderung des Wärmeleitfähigkeitssensors infolge einer Wärmeabfuhr durch das Gasgemisch zwei Messspannungen (UM3 , UM2 ) zu generieren, und die Auswerteeinheit dazu konfiguriert ist, diese zur Ermittlung von Messfehlern zu vergleicher.

2. Vorrichtung nach Anspruch 1,
**dadurch gekennzeichnet, dass**,
als Heizelement (5) ein PT 20-Sensor verwendet wird.

3. Vorrichtung nach Anspruch 1 oder 2,
**dadurch gekennzeichnet, dass**,
als Temperaturmesselement (6) ein PT 100-Sensor verwendet wird.

4. Vorrichtung nach einem der Ansprüche 1 bis 3,
**dadurch gekennzeichnet, dass**,
jeder Wärmeleitfähigkeitssensor (1) mit einem Temperatur/SpannungsWandler (θ/U-Wandler) (7) versehen ist.

5. Vorrichtung nach einem der Ansprüche 1 bis 4,
**dadurch gekennzeichnet, dass**,
der Wärmeleitfähigkeitssensor (1) einen Grundkörper (9) aufweist, auf dessen Oberseite das Heizelement (5) und das Temperatur-messelement (6) im Dünnschichtverfahren mittels Siebdruck als Leiterbahnen (10, 13) aufgebracht sind.

6. Vorrichtung nach Anspruch 5,
**dadurch gekennzeichnet, dass**,
die Leiterbahnen (10, 13) mit einem Glasüberzug bedeckt sind.

## Claims

1. Device, with a plurality of thermal conductivity sensors (1), for measuring the thermal conductivity of gas components of a gas mixture to determine the amounts of the gas components, wherein each thermal conductivity sensor (1) is part of a resistance bridge circuit and is connected to an evaluation unit belonging to the device,
wherein each thermal conductivity sensor (1) has a heating element (5) and an integrated temperature measurement element (6), **characterised in that** the heating element and the integrated temperature measurement element are configured to generate two measuring voltages (UM3, UM2) if there is a change in temperature of the thermal conductivity sensor due to removal of heat by the gas mixture, and the evaluation unit is configured to compare these to determine measuring errors.

2. Device according to claim 1,
**characterised in that** a PT 20 sensor is used as the heating element (5).

3. Device according to claim 1 or 2,
**characterised in that** a PT 100 sensor is used as the temperature measurement element (6).

4. Device according to one of claims 1 to 3,
**characterised in that** each thermal conductivity sensor (1) is fitted with a temperature/voltage transformer (θ/U transformer) (7).

5. Device according to one of claims 1 to 4,
**characterised in that** the thermal conductivity sensor (1) has a base body (9), on the top side of which the heating element (5) and the temperature measurement element (6) are applied as strip conductors (10, 13) using the thin-layer method by means of screen printing.

6. Device according to claim 5,
**characterised in that** the strip conductors (10, 13) are covered with a glass overlay.

## Revendications

1. Dispositif permettant de mesurer la conductibilité thermique de composants gazeux d'un mélange gazeux pour déterminer les fractions de composants gazeux, avec une pluralité de capteurs de conductibilité thermique (1), chaque capteur de conductibilité thermique (1) faisant partie d'un montage en pont de résistance et étant relié à une unité d'évaluation appartenant au dispositif, chaque capteur de conductibilité thermique (1) présentant un élément chauffant (5) et un élément de mesure thermique (6) intégré, **caractérisé en ce que** l'élément chauffant et l'élément de mesure thermique sont configurés pour générer deux tensions de mesure (UM3, UM2) lors d'un changement de température du capteur de conductibilité thermique dû à une dissipation de chaleur par le mélange gazeux et de comparer ces tensions de mesure pour déterminer des erreurs de mesure.

2. Dispositif selon la revendication 1, **caractérisé par** l'utilisation d'un capteur PT 20 en tant qu'élément chauffant (5).

3. Dispositif selon la revendication 1 ou la revendication 2, **caractérisé par** l'utilisation d'un capteur PT 100 en tant qu'élément de mesure thermique (6).

4. Dispositif selon l'une quelconque des revendications 1 à 3, **caractérisé en ce que** chaque capteur de conductibilité thermique (1) est pourvu d'un convertisseur température/tension (convertisseur θ/U) (7).

5. Dispositif selon l'une quelconque des revendications 1 à 4, **caractérisé en ce que** le capteur de conductibilité thermique (1) présente un corps de base (9) sur le dessus duquel l'élément chauffant (5) et l'élément de mesure thermique (6) sont appliqués par sérigraphie avec la méthode en couche mince sous forme de pistes conductrices (10, 13).

6. Dispositif selon la revendication 5, **caractérisé en ce que** les pistes conductrices (10,13) sont recouvertes d'une couche de verre.
